# EUROPEAN PATENT SPECIFICATION

(11) **EP 2 069 990 B1**
(45) Date of publication and mention of the grant of the patent: **13.03.2019**
(21) Application number: 07826421.5
(22) Date of filing: 18.09.2007
(51) Int. Cl.: G06F 19/28, G16H 50/20, G16H 50/70, G16H 10/60, G16H 70/20

(54) **A MOLECULAR DIAGNOSTICS DECISION SUPPORT SYSTEM**
ENTSCHEIDUNGSUNTERSTÜTZUNGSSYSTEM IN DER MOLEKULAREN DIAGNOSTIK
SYSTÈME DE SUPPORT DE DÉCISION DE DIAGNOSTICS MOLÉCULAIRES

(30) Priority: 20.09.2006 US 826231 P
(43) Date of publication of application: 17.06.2009
(73) Proprietor: Koninklijke Philips N.V., 5656 AE Eindhoven (NL)
(72) Inventor: ALSAFADI, Yasser, NL-5656 AE Eindhoven (NL)
(74) Representative: van Velzen, Maaike Mathilde
(86) International application number: PCT/IB2007/053762
(87) International publication number: WO 2008/035281

(56) References cited:
- WO-A-02/37398
- US-A- 5 769 074
- US-A1- 2004 122 787
- US-A1- 2004 236 723

## Description

### FIELD OF THE INVENTION

The present invention relates to a molecular diagnostics decision support system. The system can assist and/or guide a clinician, a technician or a patient during the various phases of molecular diagnostics. The invention also relates to a corresponding method.

### BACKGROUND OF THE INVENTION

Molecular diagnostics is generally recognized as the fastest-growing segment in the in-vitro diagnostics industry. The ability of molecular diagnostic (MD) assays to sensitively and specifically detect the primary cause of a disease is unprecedented.

Decision support systems for aiding with molecular diagnostics (MD) are currently applied for analyzing the values found in a molecular diagnostic assay. The result is a recommendation or guidance to a user, e.g. a clinician, who can then subsequently use this recommendation to reach a decision or an assessment with respect to the patient. Such decision support systems are quite helpful for the clinician's interpretation of values found in the molecular diagnostics when considering the amount and/or complexity of such MD data.

WO 03/017038 discloses an example of such a decision support system for aiding with molecular diagnostics (MD). The system is used for predicting which one or more drugs that are suitable for treating a cancerous condition in a patient, where the selection is based on upon the patient's genotype. A PCR kit is used for analyzing the gene expression of a patient associated with a specific cancer. Additionally, a database containing correlations between patient genotypes and the corresponding toxicity and efficacy of various anti-cancer drugs is provided. A comparison is then performed by a computerized decision support system to predict the optimum drug for the patient. Unfortunately, this type of decision support system for aiding with molecular diagnostics (MD) is unable to provide support in different stages of the health care cycle of a patient due to the rather simple approach applied. Thus, relevant clinical information from e.g. the patient's medical journal is not taken into account within the decision support system.

Another disadvantage of this type of system is the fact that the decision support system is designed and tailored to a single specific disease and this makes the system too narrow in application, and accordingly development costs are often too high compared to the result. These one-of-a-kind decision support systems therefore normally require too large investments to gain widespread use in the health sector.

Hence, an improved molecular diagnostics decision support system would be advantageous, and in particular a more efficient and/or reliable system would be advantageous.

### SUMMARY OF THE INVENTION

Accordingly, the invention preferably seeks to mitigate, alleviate or eliminate one or more of the above-mentioned disadvantages singly or in any combination. In particular, it may be seen as an object of the present invention to provide a decision support system that solves the above-mentioned problems of the prior art with performing decision support based on molecular diagnostics input.

This object and several other objects are obtained in a first aspect of the invention by providing a molecular diagnostics decision support system (MD-DSS) for providing patient related information (PRI), according to claim 1.

The invention is particularly, but not exclusively, advantageous for obtaining a decision support system with a more comprehensive approach with respect to the patient because patient data are combined with input data (MDx) derived from one or more molecular diagnostics measurement devices. Accordingly, the processing of data becomes more complicated, but using of several decision support mechanisms, the inventor has realized that advantageous results may nevertheless be provided, and in particular that decision support can be provided in more phases of the health care cycle than previously possible. This in turn can provide a comprehensive, multi-aspect view of proper current actions, thus addressing errors of commission in the health care cycle. The inclusion of clinical guidelines addresses errors of omission because the guidelines can suggest, present and/or communicate proper sequence of a care protocol.

Moreover, the present invention has the distinct advantage that several applications, e.g. several diseases or several types of users, can be integrated into one and the same decision support system. This is important because hitherto the spread of one-of-a-kind decision support systems has been limited due to the high cost of such dedicated systems.

The interaction of more than one decision support mechanism (DSM) allows processing of data streams that were previously difficult or impossible to combine into a single decision support system. The synergistic effects thereby provided facilitate a more advanced level of patient relevant information (PRI) than hitherto seen. Additionally, the combined system of the present invention can provide the patient relevant information (PRI) relatively fast.

A formal definition of what constitutes a decision support mechanism (DSM) can be very difficult to formulate. However, as will be appreciated by the skilled person examples of such mechanisms are a Bayesian network, an artificial neural network, a support vector machines mechanism, a K nearest neighbor mechanism, a genetic algorithm mechanism, a decision tree mechanism, a rule based system, a case based reasoning mechanism, and a fuzzy logic based mechanism. Thus, the first (1DSM) and/or the second (2DSM) decision support mechanism may be chosen from such a group of decision support mechanisms.

Furthermore, the decision support system according to the present invention is capable of treating multiple instantiations of a mechanism as being different as they treat different types of input data, solve different problems, or use different models or representations. For example, the decision support system may treat two instantiations of a Bayesian Network as being two distinct DSMs as they use different networks to represent the beliefs needed to solve different problems.

In one embodiment, the middleware section (MW) may comprise an application sub-section (APP), wherein an application is selectable, each application having one or more corresponding rules related to that application of the system. The decision support system may then have several applications (e.g. diseases or users), each with different rules/procedures defining the operation of the middleware. The decision support system can thereby be very adaptive.

Beneficially, the first (1DSM) and the second (2DSM) decision support mechanism may interact via one or more loose couplings, wherein a resulting output of the first decision support mechanism (1DSM) is applied as input in the second decision support mechanism (2DSM). This may take place either directly or indirectly. Alternatively or additionally, the first (1DSM) and the second (2DSM) decision support mechanism may interact via one ore more tight couplings, wherein an intermediate value of the first decision support mechanism (1DSM) is applied as input in the second decision support mechanism (2DSM). This may also take place either directly or indirectly.

Advantageously, the type of first (1DSM) and/or second (2DSM) decision support mechanism may be selected in dependency of the application. Thus, the middleware section can perform a mechanism selection. The first (1DSM) and the second (2DSM) decision support mechanism may be two different types of decision support mechanisms. This may widen the applicability of the system.

The clinical guideline database (CL) is
operably connected to communicate with a guideline execution engine (CL-EE). Similarly, the knowledge database (KB) is operably connected to communicate with an interference engine (IE). Thereby the stability of the system may be enhanced.

It is contemplated that the clinical guideline database (CL) may - at least partly - be integrated into the first decision support mechanism (1DSM) and/or the second decision support mechanism (2DSM). Likewise, the knowledge database (KB) may - at least partly - be integrated into the first decision support mechanism (1DSM) and/or the second decision support mechanism (2DSM). Thus, it may in some implementations be difficult to distinguish the clinical guideline database (CL) and/or the knowledge (KB) from the first decision support mechanism (1DSM) and/or the second decision support mechanism (2DSM).

Typically, input data (MDx) may be chosen from the group consisting of: genomic, transcriptomic, metabolonomic, epigenetic, and proteomic patterns.

In a second aspect, the present invention relates to a method for operating a molecular diagnostic decision support system (MD-DSS) for providing patient related information (PRI), according to claim 7.

In a third aspect, the invention relates to a computer program product being adapted to enable a computer system comprising at least one computer having data storage means associated therewith to control an decision support system according to the second aspect of the invention.

This aspect of the invention is particularly, but not exclusively, advantageous in that the present invention may be implemented by a computer program product enabling a computer system to perform the operations of the second aspect of the invention. Thus, it is contemplated that some known decision support may be changed to operate according to the present invention by installing a computer program product on a computer system controlling the said optical recording apparatus. Such a computer program product may be provided on any kind of computer readable medium, e.g. magnetically or optically based medium, or through a computer based network, e.g. the Internet.

The first, second and third aspect of the present invention may each be combined with any of the other aspects. These and other aspects of the invention will be apparent from and elucidated with reference to the embodiments described hereinafter.

### BRIEF DESCRIPTION OF THE FIGURES

The present invention will now be explained, by way of example only, with reference to the accompanying Figures, where
Fig. 1 is a schematic drawing of the data processing according to the present invention,
Fig. 2 is a schematic drawing illustrating the loose coupling between a first decision support mechanism (1DSM) and a second decision support mechanism (2DSM),
Fig. 3 is a schematic drawing illustrating the tight coupling between a first decision support mechanism (1DSM) and a second decision support mechanism (2DSM),
Fig. 4 is a schematic drawing of the architecture of the decision support system according to the present invention,
Fig. 5 is an embodiment of the decision support system according to the present invention assisting in determining the prognosis of a cancer disease,
Fig. 6 is a flow-chart of a method according to the invention.

### DETAILED DESCRIPTION OF AN EMBODIMENT

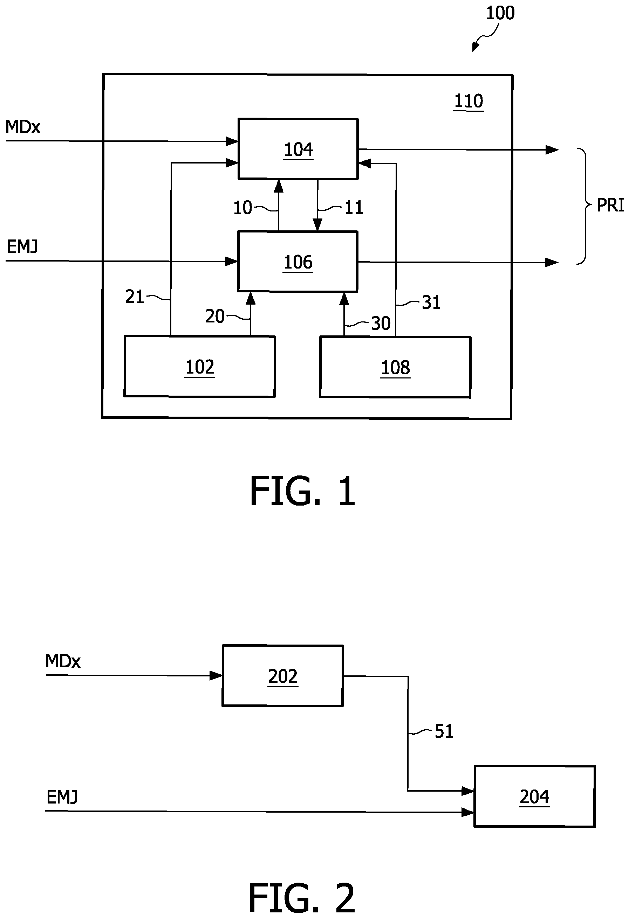

Fig. 1 is a schematic drawing of the data processing performed according to the present invention by the molecular diagnostic decision support system MD-DSS 100 for providing patient related information PRI as output.

The patient information database EMJ stores patient data about a patient, preferably in an electronic medical journal. The content of the database is related to all previously observations and actions performed on the patient. It should be mentioned that although the present invention is intended for application on a single patient at a time, it is contemplated that the invention can also be applied on a group of patients.

The data input section DAT (not shown) is adapted for receiving input data MDx about the patient from an associated molecular diagnostic measurement device (not shown).

The clinical guideline database CL 102 generally describes the course of recommended actions as will be explained in more detail below and communicates with the first decision support mechanism 1DSM 104 and the second decision support mechanism 2DSM 106 as indicated by arrows 20 and 21.

The knowledge database KB 108comprises what is necessarily or prototypically true about a domain and communicates with the first decision support mechanism 1DSM and the second decision support mechanism 2DSM as indicated by arrows 30 and 31.

The first decision support mechanism 1DSM and the second decision support mechanism 2DSM receive the input data MDx and the EMJ data, respectively. For illustrative purposes, each decision support mechanism is shown to receive only this single string of data, but likewise, each decision support mechanism 1DSM and 2DSM could receive both data strings MDx and EMJ. Additionally, it should be mentioned that the present invention is not limited to only two decision support mechanisms. Rather, it is contemplated that more than two decision support mechanisms can be applied within the teaching of the present invention.

The middleware MW section 110 interconnects the patient information database EMJ, the data input section DAT, the clinical guideline database CL, the knowledge database KB, the first decision support mechanism 1DSM, and the second decision support mechanism 2DSM. As such the other components depicted in Fig. 1 may be considered to be embedded in the middleware MW, but for practical considerations the middleware section MW enables the communication by electric/wireless connections and control this communication.

The middleware MW can therefore apply services such as directory management and security services to meet the requirements of generating decision support interventions in the data processing.

The middleware section MW is arranged for allowing input data MDx to be processed into patient related information PRI by the first 1DSM and the second 2DSM decision support mechanism using the patient information database EMJ, the clinical guideline database CL and the knowledge database KB.

The first 1DSM and the second 2DSM decision support mechanism are operably coupled, as indicated by arrows 10 and 11, through the middleware MW so as to interact during the processing of the input data MDx. By interacting it is understood that data, initial, intermediate or final in character, can be exchanged between the two decision support mechanisms 1DSM and 2DSM. Accordingly, data formats, and/or transformations therebetween, are to be in compliance with each other.

A user interface, e.g. a screen or a similar device, for presenting the patient related information PRI should be used. The user interface can be specific according to the application.

It is to be understood that the present invention does not provide a diagnosis as part of the patient related information PRI. Rather, the invention provides information PRI that can assist a physician, a clinician, and/or a technician in reaching a diagnosis.

The invention as illustrated in Fig. 1 can be implemented in any suitable form including hardware, software, firmware or any combination of these. The invention or some features of the invention can be implemented as computer software running on one or more data processors and/or digital signal processors. The elements and components of an embodiment of the invention may be physically, functionally and logically implemented in any suitable way. Indeed, the functionality may be implemented in a single unit, in a plurality of units or as part of other functional units. As such, the invention may be implemented in a single unit, or may be physically and functionally distributed between different units and processors. The middleware section MW interconnecting the elements of the DSS can include any kind of connection including, but not limited to, electrical connections and wireless connections. In particular, wireless connections can facilitate that patient related information PRI is provided at the point of care for the patient.

Fig. 2 is a schematic drawing illustrating the loose coupling 51 between a first decision support mechanism 1DSM 202 and a second decision support mechanism 2DSM 204. By loose coupling it is to be understood that a resulting output of the first decision support mechanism 1DSM is applied as input in the second decision support mechanism 2DSM as indicated by the arrow 51. This can take place either directly or indirectly depending on the kind of coupling. In short, the two mechanisms are treated as "black boxes" where output of one mechanism is coupled with input of another mechanism. The decision support mechanisms have to agree on the format and semantics of output/input coupling, but they do not interfere with the internal working of the other mechanism.

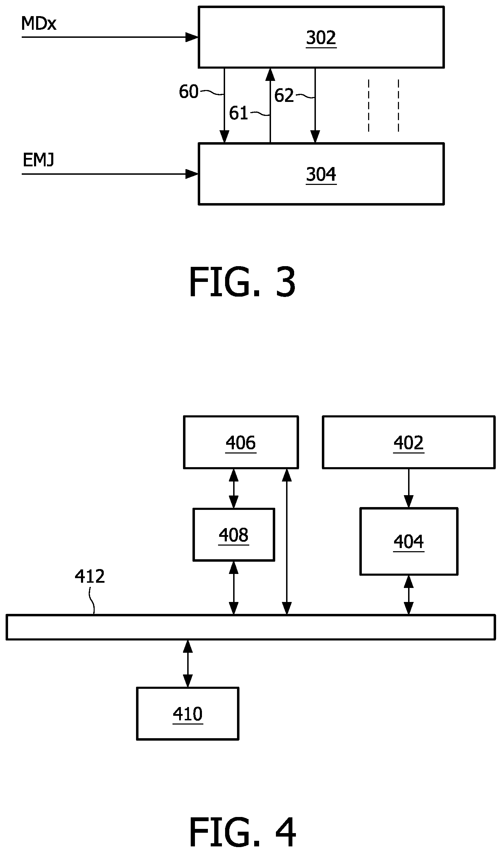

Fig. 3 is a schematic drawing illustrating the tight couplings 60, 61, and 62 between a first decision support mechanism 1DSM 302 and a second decision support mechanism 2DSM 304. An intermediate value of the first decision support mechanism 1DSM is applied as input in the second decision support mechanism 2DSM. Again, this can take place either directly or indirectly as mentioned above. In that respect, mechanisms 1DSM and 2DSM are treated as "open boxes". They interact tightly from within the mechanism. One example is to use inferences from the Knowledge Base KB to drive the crossover and mutations in a Genetic Algorithm (GA). This tight coupling will introduce biological knowledge to guide the purely statistical process in the Genetic Algorithm GA. Thus, the genes in the GA are not treated as anonymous independent entities, but rather as ones with significance and interrelationships. Another example is to use a Bayesian - or a Fuzzy logic based network to resolve a criterion in a decision step in a clinical guideline CL. This requires the Bayesian/Fuzzy network to access the patient state in the guideline.

Fig. 4 is a schematic drawing of the architecture of the decision support system according to the present invention. In addition to the components shown in Fig. 1, the clinical guideline database CL 402 is operably connected to communicate with a guideline execution engine CL-EE 404, and likewise the knowledge database KB 406 is operably connected to communicate with an interference engine IE 408.

The guideline execution engine CL-EE and inference engine IE are generic components of a decision support system and have no specific requirements for a molecular diagnosis application. The guideline execution engine CL-EE is a software component that can step through the executable guideline and suggest one or more steps in the guideline based on patient status. The GLIF3 Guideline Execution Engine (GLEE) is an example of such engine.

The inference engine IE will classify the asserted facts in the knowledge base KB to find the correct position of a class in a hierarchy using the logical definition of the classes. This will enable an application section to learn about the inferred relationships among the facts. Racer is an example inference engine. It should be consider to use available off the shelf components for these engines IE and CL-EE.

With respect to guidelines CL, it can be said that some guidelines aim to overcome the problem of having a limited number of trained technicians who are capable of performing complex assays. Other guidelines aim to identify the necessary education and counseling that must be made available to patients. For example the Huntington's disease Society of America provides a series of guides aimed at educating and counseling patients and their families.

The guidelines component can be a repository of guidelines. Machine processable guidelines can be represented in the GuideLine Interchange Format GLIF 3.5. Guidelines meant to be rendered on screens to be read by humans can be represented in Guideline Elements Model (GEM), see GLIF 3.5 Technical Specification, Draft Release, InterMed Collaboratory, December 12, 2002.

The selection of a representation format depends on the intended uses of a guideline in the system. Executable guidelines are very powerful as they can be used to monitor the progress of clinical care. GEM-like encoded guidelines are useful when presenting relevant knowledge and evidence for users to read and consider. System designers should be aware of the effort needed to represent paper guidelines, similar to the ones previously discussed, in an electronic format.

With respect to knowledge database KB, it can be said that ontologies can comprise an essential part of such a database. Ontologies offer a conceptualization of domains of knowledge and facilitate both communication between researchers and the use of domain knowledge by computers. For example, the Gene Ontology (GO) project aims to provide consistent descriptors for gene products, in different databases; and to standardize classifications for sequences and sequence features. GO allows annotating genes and their products with a limited set of attributes. A gene product has one or more molecular functions and is used in one or more biological processes. However, GO does not specify where or when, or in what context the action takes place. This knowledge is captured by Reactome, which is a curated peer-reviewed knowledge-base of human biological process. Another relevant source is dbSNP, a database for human gene mutations.

The knowledge bases can be used when handling patient data. Molecular tests generate granular findings that need to be saved as part of the patient record. An example of a general clinical knowledge base that can be used to derive descriptive findings is SNOMED Clinical Terms (SNOMED-CT®) ontology. However it lacks the concepts appropriate for the detailed description of chromosomal structures and lacks the terms needed for accurate description of molecular findings related to gene mutations and polymorphisms. The Clinical Bioinformatics Ontology™ (CBO) aims to fill this gap between general clinical knowledge and detailed molecular findings.

Finally, there are two concerns in using these knowledge bases KB. Firstly, different knowledge bases KB use different identifiers to track genes and their products. System designers should therefore consider solutions when the conversion from one type of identifiers to another is not a one-to-one relationship. Secondly, in this emerging field, some holes exist in the understanding of biological pathways. Those holes should be identified according to the knowledge needs of the decisions to be made. Bioinformatics methods can suggest items to fill these holes. Consequently, these suggestions should be experimentally verified.

With respect to the patient information database EMJ, it could be said that the decision support system MD DSS will include patient specific data to personalize the generated decisions. A patient's molecular profile can summarize the past results of molecular tests. A detailed family history can be important and often suggests a genetic predisposition. Drug history should be considered to identify possible drugs that may be contributing to some symptoms. Patient data may include imaging measurements that quantitatively correlate (either directly or inversely) with disease progression. Laboratory data can contain measurements from previous molecular tests and traditional tests that can establish a proper history to monitor the progress of a disease. The database can include information about the specific preferences and values that the patient holds. A knowledge-based approach is important to integrate these different sources of patient data.

Since molecular diagnosis is an emerging field it is not always certain that the electronic medical record EMJ 410 will include a critical mass of observations such that it is able to answer the data needs of a molecular diagnostics decision support system MD DSS. It has to be decided what recommendations the decision support system MD DSS needs to produce, and work backwards to establish what data must be collected in order to support the production of these recommendations, and further backwards to identify where the relevant data come from and how they will be captured and normalized, see S. Coady, Influencing physician practice variation, in Health Management Technology, 2002.

The EMJ is operably connected to the Interference engine and the clinical guideline execution engine through middleware MW 412.

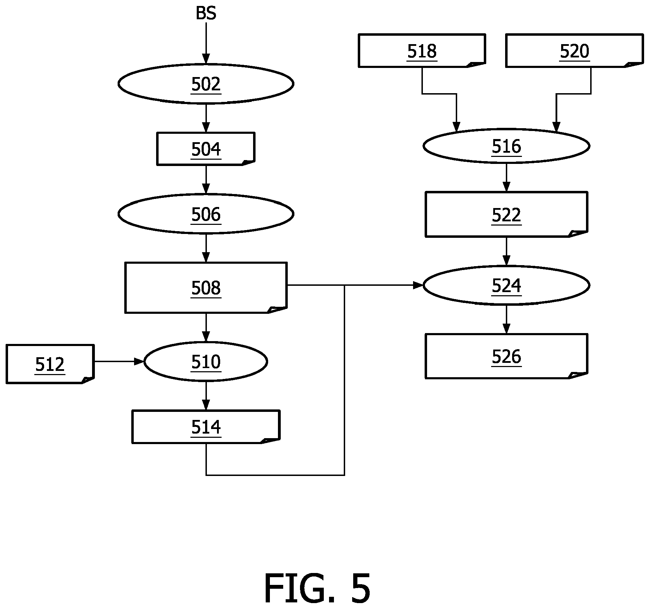

Fig. 5 is an embodiment of the decision support system according to the present invention assisting in determining the prognosis of a cancer disease. Prognosis is an important step towards therapy planning. The clinician faces considerable uncertainty in terms of predicting what will happen to an individual patient, and what the survival probability is. In the case of a tumor, what is the chance of going to metastasis, what would be the metastatic pattern, and what is the survival time and so forth. Prognosis affects the selection of therapy for a particular patient.

The measurement device MD 502, using e.g. cDNA microarrays, can provide the gene expression of biopsy samples BS, thus molecular diagnostic input data MDx 504 is provided.

A Bayesian Network 1DSM/1BAY 506 is constructed, based on clinical trials and outcomes of many patients, to capture the probability of membership in one of a number of cancer subgroups. An example of cancer subgroups could be aggressive versus non-aggressive breast cancer. Based on the cDNA data, the Bayesian Network estimates the patient's cancer subgroup EST 508.

Based on this membership information, the interference engine IE 510 traverses a knowledge base KB 512 about cancers and their metastatic patterns MP 514 and extracts the corresponding patterns and the associated survival rates.

In parallel, the clinical guideline execution engine CL-EE 516 selects the clinical guideline 518 that the patient is enrolled in using the electronic medical record EMJ 520 and indicates the previous, current and suggested treatment steps STEP 522 for this patient.

Another Bayesian network 2DSM/2BAY 524 is constructed to generate the probability of survival PROG based on different treatment alternatives, estimates of cancer subgroup memberships, and metastatic patterns. The results from the decision support mechanism 1DSM/1BAY is feed into the other Bayesian network 2DSM/2BAY to provide the prognosis PROG 526 as depicted in Fig. 5.

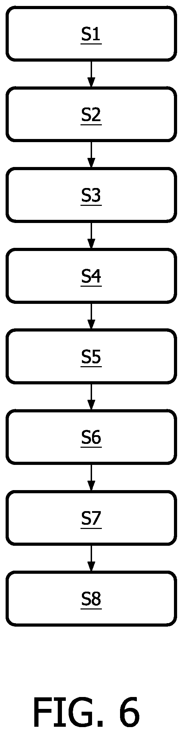

Fig. 6 is a flow-chart of a method according to the invention. The steps of the method do not necessarily have to be performed in the listed sequence below as it will be understood by the person skilled in the art. The method comprises the steps of:
- S1 providing patient data about a patient stored on a patient information database EMJ,
- S2 receiving input data MDx about the patient from a molecular diagnostics measurement device in a data input section DAT,
- S3 providing a clinical guideline database CL,
- S4 providing a knowledge database KB,
- S5 providing a first decision support mechanism 1DSM,
- S6 providing a second decision support mechanism 2DSM,
- S7 providing a middleware MW section for interconnecting the patient information database EMJ, the data input section DAT, the clinical guideline database CL, the knowledge database KB, the first decision support mechanism 1DSM, and the second decision support mechanism 2DSM, and
- S8 processing the input data MDx into patient related information PRI by the first 1DSM and the second 2DSM decision support mechanism using the patient information database EMJ, the clinical guideline database CL and the knowledge database KB,
wherein the first 1DSM and the second 2DSM decision support mechanism are operably coupled through the middleware MW so as to interact 10, 11, 51, 60, 61 or 62 during the processing of the input data MDx.

Although the present invention has been described in connection with the specified embodiments, it is not intended to be limited to the specific form set forth herein. Rather, the scope of the present invention is limited only by the accompanying claims. In the claims, the term "comprising" does not exclude the presence of other elements or steps. Additionally, although individual features may be included in different claims, these may possibly be advantageously combined, and the inclusion in different claims does not imply that a combination of features is not feasible and/or advantageous. In addition, singular references do not exclude a plurality. Thus, references to "a", "an", "first", "second" etc. do not preclude a plurality. Furthermore, reference signs in the claims shall not be construed as limiting the scope.

## Claims

1. A molecular diagnostics decision support system (MD-DSS) for providing patient related information (PRI), the system comprising:
- a patient information database (EMJ) storing patient data about a patient,
- a data input section (DAT) adapted for receiving input data (MDx) about the patient from an associated molecular diagnostic measurement device, the input data (MDx) comprising data chosen from the group consisting of: genomic, transcriptomic, metabolonomic, epigenetic, and proteomic patterns,
- a clinical guideline database (CL) operably connected to communicate with a guideline execution engine (CL-EE),
- a knowledge database (KB) operably connected to communicate with an inference engine (IE), the knowledge database (KB) comprising an ontology,
- a first decision support mechanism (1DSM),
- a second decision support mechanism (2DSM), and
- a middleware (MW) section interconnecting the patient information database (EMJ), the data input section (DAT), the clinical guideline database (CL), the knowledge database (KB), the first decision support mechanism (1DSM), and the second decision support mechanism (2DSM),
wherein the middleware section is arranged for allowing input data (MDx) to be processed into patient related information (PRI) by the first (1DSM) and the second (2DSM) decision support mechanism using the patient information database (EMJ), the clinical guideline database (CL) and the knowledge database (KB),
wherein the first (1DSM) and the second (2DSM) decision support mechanism are operably coupled through the middleware (MW) so as to interact (10, 11, 51, 60, 61, 62) during the processing of the input data (MDx);
wherein the first (1DSM) and the second (2DSM) decision support mechanism interact via one or more loose couplings (51) and/or via one or more tight couplings (60, 61, 62), wherein via a loose coupling (51), a resulting output of the first decision support mechanism (1DSM) is applied is as input in the second decision support mechanism (2DSM) and, via a tight coupling (60, 61, 62), an intermediate value of the first decision support mechanism (1DSM) is applied is as input in the second decision support mechanism (2DSM); and
wherein the first (1DSM) and/or the second (2DSM) decision support mechanism is chosen from the group consisting of: a Bayesian network, an artificial neural network, a support vector machines mechanism, a K nearest neighbor mechanism, a genetic algorithm mechanism, a decision tree mechanism, a rule based system, a case based reasoning mechanism, and a fuzzy logic based mechanism.

2. A system according to claim 1, wherein the middleware section (MW) comprises an application sub-section (APP), wherein an application is selectable, each application having one or more corresponding rules related to that application of the system.

3. A system according to claim 1, wherein the type of the first (1DSM) and/or second (2DSM) decision support mechanism is selected in dependency of the application.

4. A system according to claim 1, wherein the first (1DSM) and the second (2DSM) decision support mechanism are two different types of decision support mechanisms.

5. A system according to claim 1, wherein the clinical guideline database (CL) is - at least partly - integrated into the first decision support mechanism (1DSM) and/or the second decision support mechanism (2DSM).

6. A system according to claim 1, wherein the knowledge database (KB) is - at least partly - integrated into the first decision support mechanism (1DSM) and/or the second decision support mechanism (2DSM).

7. A method for operating a molecular diagnostics decision support system (MD-DSS) for providing patient related information (PRI), the method comprises the steps of:
- providing patient data about a patient stored in a patient information database (EMJ),
- receiving input data (MDx) about the patient from a molecular diagnostics measurement device in a data input section (DAT), the input data (MDx) comprising data chosen from the group consisting of: genomic, transcriptomic, metabolonomic, epigenetic, and proteomic patterns,
- providing a clinical guideline database (CL) operably connected to communicate with a guideline execution engine (CL-EE),
- providing a knowledge database (KB) operably connected to communicate with an inference engine (IE), the knowledge database (KB) comprising an ontology,
- providing a first decision support mechanism (1DSM),
- providing a second decision support mechanism (2DSM),
- providing a middleware (MW) section for interconnecting the patient information database (EMJ), the data input section (DAT), the clinical guideline database (CL), the knowledge database (KB), the first decision support mechanism (1DSM), and the second decision support mechanism (2DSM), and
- processing the input data (MDx) into patient related information (PRI) by the first (1DSM) and the second (2DSM) decision support mechanism using the patient information database (EMJ), the clinical guideline database (CL) and the knowledge database (KB),
wherein the first (1DSM) and the second (2DSM) decision support mechanism are operably coupled through the middleware (MW) so as to interact (10, 11, 51, 60, 61, 62) during the processing of the input data (MDx);
wherein the first (1DSM) and the second (2DSM) decision support mechanism interact via one or more loose couplings (51) and/or via one or more tight couplings (60, 61, 62), wherein via a loose coupling (51), a resulting output of the first decision support mechanism (1DSM) is applied is as input in the second decision support mechanism (2DSM) and, via a tight coupling (60, 61, 62), an intermediate value of the first decision support mechanism (1DSM) is applied is as input in the second decision support mechanism (2DSM); and
wherein the first (1DSM) and/or the second (2DSM) decision support mechanism is chosen from the group consisting of: a Bayesian network, an artificial neural network, a support vector machines mechanism, a K nearest neighbor mechanism, a genetic algorithm mechanism, a decision tree mechanism, a rule based system, a case based reasoning mechanism, and a fuzzy logic based mechanism..

8. A computer program product being adapted to enable a computer system comprising at least one computer having data storage means associated therewith to control a decision support system according to the method of claim 7.

## Patentansprüche

1. Entscheidungsunterstützungssystem in der molekularen Diagnostik (MD-DSS) zur Bereitstellung von patientenbezogenen Informationen (PRI), wobei das System Folgendes umfasst:
- eine Patienteninformationsdatenbank (EMJ), in der Patientendaten über einen Patienten gespeichert sind,
- einen Dateneingabeabschnitt (DAT), der dafür ausgelegt ist, Eingabedaten (MDx) über den Patienten von einer zugehörigen molekulardiagnostischen Messvorrichtung zu empfangen, wobei die Eingabedaten (MDx) Daten ausgewählt aus der Gruppe bestehend aus genomischen, transkriptomischen, metabolonomischen, epigenetischen und proteomischen Mustern umfassen,
- eine klinische Richtliniendatenbank (CL), die betriebsfähig verbunden ist, um mit einer Richtlinienausführungsmaschine (CL-EE) zu kommunizieren,
- eine Wissensdatenbank (KB), die betriebsfähig verbunden ist, um mit einer Inferenzmaschine (IE) zu kommunizieren, wobei die Wissensdatenbank (KB) eine Ontologie umfasst,
- einen ersten Entscheidungsunterstützungsmechanismus (1DSM),
- einen zweiten Entscheidungsunterstützungsmechanismus (2DSM) und
- einen Middleware (MW)-Abschnitt, der die Patienteninformationsdatenbank (EMJ), den Dateneingabeabschnitt (DAT), die klinische Richtliniendatenbank (CL), die Wissensdatenbank (KB), den ersten Entscheidungsunterstützungsmechanismus (1DSM) und den zweiten Entscheidungsunterstützungsmechanismus (2DSM) miteinander verbindet,
wobei der Middleware-Abschnitt dafür eingerichtet ist, die Verarbeitung der Eingabedaten (MDx) zu patientenbezogenen Informationen (PRI) durch den ersten (1DSM) und den zweiten (2DSM) Entscheidungsunterstützungsmechanismus unter Verwendung der Patienteninformationsdatenbank (EMJ), der klinischen Richtliniendatenbank (CL) und der Wissensdatenbank (KB) zu erlauben,
wobei der erste (1DSM) und der zweite (2DSM) Entscheidungsunterstützungsmechanismus betriebsfähig über die Middleware (MW) gekoppelt sind, um so während der Verarbeitung der Eingabedaten (MDx) zu interagieren (10, 11, 51, 60, 61, 62);
wobei der erste (1DSM) und der zweite (2DSM) Entscheidungsunterstützungsmechanismus über eine oder mehrere lockere Kopplungen (51) und/oder über eine oder mehrere enge Kopplungen (60, 61, 62) interagieren, wobei über eine lockere Kopplung (51) eine resultierende Ausgabe des ersten Entscheidungsunterstützungsmechanismus (1DSM) als Eingabe in den zweiten Entscheidungsunterstützungsmechanismus (2DSM) angewendet wird und über eine enge Kopplung (60, 61, 62) ein Zwischenwert des ersten Entscheidungsunterstützungsmechanismus (1DSM) als Eingabe in den zweiten Entscheidungsunterstützungsmechanismus (2DSM) angewendet wird; und
wobei der erste (1DSM) und/oder der zweite (2DSM) Entscheidungsunterstützungsmechanismus ausgewählt sind aus der Gruppe bestehend aus: einem Bayes'schen Netzwerk, einem künstlichen neuralen Netzwerk, einem Unterstützungsvektormaschinenmechanismus, einem K-nächste-Nachbarn-Mechanismus, einem genetischen Algorithmusmechanismus, einem Entscheidungsbaummechanismus, einem regelbasierten System, einem fallbasierten Schlussfolgerungsmechanismus und einem Fuzzy-Logik-basierten Mechanismus.

2. System nach Anspruch 1, wobei der Middleware-Abschnitt (MW) einen Anwendungs-Teilabschnitt (APP) umfasst, in dem eine Anwendung auswählbar ist, wobei jede Anwendung eine oder mehrere entsprechende Regeln in Bezug auf diese Anwendung des Systems hat.

3. System nach Anspruch 1, wobei die Art des ersten (1DSM) und/oder des zweiten (2DSM) Entscheidungsunterstützungsmechanismus in Abhängigkeit von der Anwendung ausgewählt wird.

4. System nach Anspruch 1, wobei der erste (1DSM) und der zweite (2DSM) Entscheidungsunterstützungsmechanismus zwei verschiedene Arten von Entscheidungsunterstützungsmechanismen sind.

5. System nach Anspruch 1, wobei die klinische Richtliniendatenbank (CL) - zumindest teilweise - in den ersten Entscheidungsunterstützungsmechanismus (1DSM) und/oder den zweiten Entscheidungsunterstützungsmechanismus (2DSM) integriert ist.

6. System nach Anspruch 1, wobei die Wissensdatenbank (KB) - zumindest teilweise - in den ersten Entscheidungsunterstützungsmechanismus (1DSM) und/oder den zweiten Entscheidungsunterstützungsmechanismus (2DSM) integriert ist.

7. Verfahren zum Betreiben eines Entscheidungsunterstützungssystems in der molekularen Diagnostik (MD-DSS) zur Bereitstellung von patientenbezogenen Informationen (PRI), wobei das Verfahren die folgenden Schritte umfasst:
- Bereitstellen von Patientendaten über einen Patienten, die in einer Patienteninformationsdatenbank (EMJ) gespeichert sind,
- Empfangen von Eingabedaten (MDx) über den Patienten von einer molekulardiagnostischen Messvorrichtung in einem Dateneingabeabschnitt (DAT), wobei die Eingabedaten (MDx) Daten ausgewählt aus der Gruppe bestehend aus genomischen, transkriptomischen, metabolonomischen, epigenetischen und proteomischen Mustern umfassen,
- Bereitstellen einer klinischen Richtliniendatenbank (CL), die betriebsfähig verbunden ist, um mit einer Richtlinienausführungsmaschine (CL-EE) zu kommunizieren,
- Bereitstellen einer Wissensdatenbank (KB), die betriebsfähig verbunden ist, um mit einer Inferenzmaschine (IE) zu kommunizieren, wobei die Wissensdatenbank (KB) eine Ontologie umfasst,
- Bereitstellen eines ersten Entscheidungsunterstützungsmechanismus (1DSM),
- Bereitstellen eines zweiten Entscheidungsunterstützungsmechanismus (2DSM) und
- Bereitstellen eines Middleware (MW)-Abschnitts zum Verbinden der Patienteninformationsdatenbank (EMJ), des Dateneingabeabschnitt (DAT), der klinischen Richtliniendatenbank (CL), der Wissensdatenbank (KB), des ersten Entscheidungsunterstützungsmechanismus (1DSM) und des zweiten Entscheidungsunterstützungsmechanismus (2DSM) miteinander, und
- Verarbeiten der Eingabedaten (MDx) zu patientenbezogenen Informationen (PRI) durch den ersten (1DSM) und den zweiten (2DSM) Entscheidungsunterstützungsmechanismus unter Verwendung der Patienteninformationsdatenbank (EMJ), der klinischen Richtliniendatenbank (CL) und der Wissensdatenbank (KB),
wobei der erste (1DSM) und der zweite (2DSM) Entscheidungsunterstützungsmechanismus betriebsfähig über die Middleware (MW) gekoppelt sind, um so während der Verarbeitung der Eingabedaten (MDx) zu interagieren (10, 11, 51, 60, 61, 62);
wobei der erste (1DSM) und der zweite (2DSM) Entscheidungsunterstützungsmechanismus über eine oder mehrere lockere Kopplungen (51) und/oder über eine oder mehrere enge Kopplungen (60, 61, 62) interagieren, wobei über eine lockere Kopplung (51) eine resultierende Ausgabe des ersten Entscheidungsunterstützungsmechanismus (1DSM) als Eingabe in den zweiten Entscheidungsunterstützungsmechanismus (2DSM) angewendet wird und über eine enge Kopplung (60, 61, 62) ein Zwischenwert des ersten Entscheidungsunterstützungsmechanismus (1DSM) als Eingabe in den zweiten Entscheidungsunterstützungsmechanismus (2DSM) angewendet wird; und
wobei der erste (1DSM) und/oder der zweite (2DSM) Entscheidungsunterstützungsmechanismus ausgewählt sind aus der Gruppe bestehend aus: einem Bayes'schen Netzwerk, einem künstlichen neuralen Netzwerk, einem Unterstützungsvektormaschinenmechanismus, einem K-nächste-Nachbarn-Mechanismus, einem genetischen Algorithmusmechanismus, einem Entscheidungsbaummechanismus, einem regelbasierten System, einem fallbasierten Schlussfolgerungsmechanismus und einem Fuzzy-Logik-basierten Mechanismus.

8. Computerprogrammprodukt, das dafür ausgelegt ist, ein Computersystem, das mindestens einen Computer mit zugehörigen Datenspeichermitteln umfasst, zu befähigen, ein Entscheidungsunterstützungssystem entsprechend dem Verfahren aus Anspruch 7 zu steuern.

## Revendications

1. Système d'aide à la décision pour diagnostic moléculaire (MD-DSS) pour fournir des informations liées à un patient (PRI), le système comprenant :
- une base de données d'informations de patient (EMJ) stockant des données de patient concernant un patient,
- une section d'entrée de données (DAT) conçue pour recevoir des données d'entrée (MDx) concernant le patient en provenance d'un dispositif de mesure de diagnostic moléculaire associé, les données d'entrée (MDx) comprenant des données choisies dans le groupe constitué par : des modèles génomiques, transcriptomiques, métabolomiques, épigénétiques et protéomiques,
- une base de données de directives cliniques (CL) reliée de manière opérationnelle pour communiquer avec un moteur d'exécution de directive (CL-EE),
- une base de données de connaissances (KB) reliée de manière opérationnelle pour communiquer avec un moteur d'inférence (IE), la base de données de connaissances (KB) comprenant une ontologie,
- un premier mécanisme d'aide à la décision (1DSM),
- un second mécanisme d'aide à la décision (2DSM), et
- une section de logiciel médiateur (MW) interconnectant la base de données d'informations de patient (EMJ), la section d'entrée de données (DAT), la base de données de directives cliniques (CL), la base de données de connaissances (KB), le premier mécanisme d'aide à la décision (1DSM) et le second mécanisme d'aide à la décision (2DSM),
dans lequel la section de logiciel médiateur est agencée pour permettre à des données d'entrée (MDx) d'être traitées en informations liées à un patient (PRI) par le premier (1DSM) et le second (2DSM) mécanisme d'aide à la décision en utilisant la base de données d'informations de patient (EMJ), la base de données de directives cliniques (CL) et la base de données de connaissances (KB),
dans lequel le premier (1DSM) et le second (2DSM) mécanisme d'aide à la décision sont reliés de manière opérationnelle par le logiciel médiateur (MW) afin d'interagir (10, 11, 51, 60, 61, 62) pendant le traitement des données d'entrée (MDx) ;
dans lequel le premier (1DSM) et le second (2DSM) mécanisme d'aide à la décision interagissent via un ou plusieurs couplages lâches (51) et/ou via un ou plusieurs couplages étroits (60, 61, 62), dans lequel via un couplage lâche (51), une sortie résultante du premier mécanisme d'aide à la décision (1DSM) est appliquée est comme une entrée dans le second mécanisme d'aide à la décision (2DSM) et, via un couplage étroit (60, 61, 62), une valeur intermédiaire du premier mécanisme d'aide à la décision (1DSM) est appliquée est comme une entrée dans le second mécanisme d'aide à la décision (2DSM) ; et
dans lequel le premier (1DSM) et/ou le second (2DSM) mécanisme d'aide à la décision sont choisis dans le groupe constitué par : un réseau bayésien, un réseau neuronal artificiel, un mécanisme de machines vectorielles d'aide, un mécanisme de voisin K le plus proche, un mécanisme d'algorithme génétique, un mécanisme d'arbre de décision, un système à base de règles, un mécanisme de raisonnement à base de cas et un mécanisme à base de logique floue.

2. Système selon la revendication 1, dans lequel la section de logiciel médiateur (MW) comprend une sous-section d'application (APP), dans lequel une application peut être sélectionnée, chaque application ayant une ou plusieurs règles correspondantes liées à cette application du système.

3. Système selon la revendication 1, dans lequel le type du premier (1DSM) et/ou du second (2DSM) mécanisme d'aide à la décision est sélectionné en fonction de l'application.

4. Système selon la revendication 1, dans lequel le premier (1DSM) et le second (2DSM) mécanisme d'aide à la décision sont deux types différents de mécanismes d'aide à la décision.

5. Système selon la revendication 1, dans lequel la base de données de directives cliniques (CL) est - au moins en partie - intégrée dans le premier mécanisme d'aide à la décision (1DSM) et/ou le second mécanisme d'aide à la décision (2DSM).

6. Système selon la revendication 1, dans lequel la base de données de connaissances (KB) est - au moins en partie - intégrée dans le premier mécanisme d'aide à la décision (1DSM) et/ou le second mécanisme d'aide à la décision (2DSM).

7. Procédé pour mettre en oeuvre un système d'aide à la décision pour diagnostic moléculaire (MD-DSS) pour fournir des informations liées à un patient (PRI), le procédé comprend les étapes de :
- fourniture de données de patient concernant un patient stockées dans une base de données d'informations de patient (EMJ),
- réception de données d'entrée (MDx) concernant le patient à partir d'un dispositif de mesure de diagnostic moléculaire dans une section d'entrée de données (DAT), les données d'entrée (MDx) comprenant des données choisies dans le groupe constitué par : des modèles génomiques, transcriptomiques, métabolomiques, épigénétiques et protéomiques,
- fourniture d'une base de données de directives cliniques (CL) reliée de manière opérationnelle pour communiquer avec un moteur d'exécution de directive (CL-EE),
- fourniture d'une base de données de connaissances (KB) reliée de manière opérationnelle pour communiquer avec un moteur d'inférence (IE), la base de données de connaissances (KB) comprenant une ontologie,
- fourniture d'un premier mécanisme d'aide à la décision (1DSM),
- fourniture d'un second mécanisme d'aide à la décision (2DSM),
- fourniture d'une section de logiciel médiateur (MW) pour interconnecter la base de données d'informations de patient (EMJ), la section d'entrée de données (DAT), la base de données de directives cliniques (CL), la base de données de connaissances (KB), le premier mécanisme d'aide à la décision (1DSM) et le second mécanisme d'aide à la décision (2DSM), et
- traitement des données d'entrée (MDx) en informations liées à un patient (PRI) par le premier (1DSM) et le second (2DSM) mécanisme d'aide à la décision en utilisant la base de données d'informations de patient (EMJ), la base de données de directives cliniques (CL) et la base de données de connaissances (KB),
dans lequel le premier (1DSM) et le second (2DSM) mécanisme d'aide à la décision sont reliés de manière opérationnelle par le logiciel médiateur (MW) afin d'interagir (10, 11, 51, 60, 61, 62) pendant le traitement des données d'entrée (MDx) ;
dans lequel le premier (1DSM) et le second (2DSM) mécanisme d'aide à la décision interagissent via un ou plusieurs couplages lâches (51) et/ou via un ou plusieurs couplages étroits (60, 61, 62), dans lequel via un couplage lâche (51), une sortie résultante du premier mécanisme d'aide à la décision (1DSM) est appliquée est comme une entrée dans le second mécanisme d'aide à la décision (2DSM) et, via un couplage étroit (60, 61, 62), une valeur intermédiaire du premier mécanisme d'aide à la décision (1DSM) est appliquée est comme une entrée dans le second mécanisme d'aide à la décision (2DSM) ; et
dans lequel le premier (1DSM) et/ou le second (2DSM) mécanisme d'aide à la décision sont choisis dans le groupe constitué par : un réseau bayésien, un réseau neuronal artificiel, un mécanisme de machines vectorielles d'aide, un mécanisme de voisin K le plus proche, un mécanisme d'algorithme génétique, un mécanisme d'arbre de décision, un système à base de règles, un mécanisme de raisonnement à base de cas et un mécanisme à base de logique floue.

8. Produit formant programme informatique qui est conçu pour permettre à un système informatique comprenant au moins un ordinateur ayant un moyen de stockage de données associé à celui-ci de commander un système d'aide à la décision selon le procédé de la revendication 7.
